# EUROPÄISCHE PATENTSCHRIFT

(11) **EP 0 979 053 B1**
(45) Veröffentlichungstag und Bekanntmachung des Hinweises auf die Patenterteilung: **29.12.2004**
(21) Anmeldenummer: 98920541.4
(22) Anmeldetag: 21.04.1998
(51) Int. Cl.: A61B 5/00

(54) **TRAGBARES, NETZUNABHÄNGIGES MESS- UND DIAGNOSESYSTEM**
PORTABLE, NONSYSTEM-CONNECTED MEASURING AND DIAGNOSTIC SYSTEM
SYSTEME DE MESURE ET DE DIAGNOSTIC PORTATIF AUTONOME

(30) Priorität: 21.04.1997 DE 29707141 U; 12.08.1997 DE 29714445 U; 19.08.1997 DE 29714826 U
(43) Veröffentlichungstag der Anmeldung: 16.02.2000
(73) Patentinhaber: Schneider, Edgar, 85386 Eching (DE)
(72) Erfinder: Schneider, Edgar, 85386 Eching (DE)
(86) Internationale Anmeldenummer: PCT/EP1998/002339
(87) Internationale Veröffentlichungsnummer: WO 1998/047423

(56) Entgegenhaltungen:
- WO-A-89/00024
- WO-A-93/06776
- WO-A-94/13198
- DE-A- 3 234 884

## Beschreibung

Die Erfindung betrifft ein tragbares netzunabhängiges Meß- und Diagnosesystem gemäß Anspruch 1, das insbesondere als medizinisches Meß- und Diagnosesystem genutzt werden kann.

In der Medizin sind heute eine Vielzahl von unterschiedlichsten Meß- und Diagnosesystemen und Geräten im Einsatz. Für den einzelnen Arzt ist es äußerst schwierig bzw. nahezu unmöglich, die Bedienung der Vielzahl von verschiedenen Geräten zu beherrschen. Er beschränkt sich daher notgedrungen auf eine kleinere Auswahl von Geräten, was dazu führen kann, daß in einem bestimmten Fall ein geeignetes Meß- und Diagnosegerät nicht angewandt wird.

Aus der WO96/14014 ist bereits ein tragbares medizinisches Meß- und Diagnosegerät bekannt, bei dem in eine kompakte Bauform neben einer Ultraschallmeßeinrichtung verschiedene andere Meßeinrichtungen für physiologische Werte, wie Temperatur und EKG integriert sind. Nachteilig bei diesem bekannten medizinischen Meß- und Diagnosegerät ist, daß nur unterschiedliche Ultraschallmeßeinrichtungen mit dem Grundgerät verbindbar sind.

Im Bereich der Umwelt- und Schadstoffmeßtechnik sind je nach Verwendungszweck eine Vielzahl von unterschiedlichen Meßgeräten im Einsatz. Um unterschiedliche Schadstoffe zu erfassen bzw. für Messungen in verschiedenen Medien (Luft, Wasser, Erdreich etc.) sind unterschiedlichste Meßgeräte und Systeme notwendig.

Aus WO-A-93/06776 ist eine stationäres Meß- und Diagnosesystem bekannt, bei dem unterschiedliche Meßkatheter mit einem Grundmodul verbunden werden und wobei die Meßkatheter eine spezifische Kennnung aufweisen. Diese spezifische Kennung ist in einem Speicherelement in dem jeweiligen Meßkatheter abgespeichert und umfaßt Herstellerdaten, Seriennummer, Patientendaten usw. Auf diese Weise wird verhindert, daß an sich funkionsfähige "Fremdkatheter" an dem System betrieben werden können. Zusätzlich kann die spezifische Kennung auch Kalibrierungsdaten umfaßen, so daß die gemessenen Daten automatisch richtg kalibriert werden.

Aus der WO-A-94/13189 ist ein tragbares, netzunabhängiges Meß- und Diagnosesystem nach dem Oberbegriff des Anspruchs 1 bekannt, das ein Grundmodul und eine Mehrzahl damit lösbar verbindbarer Meßmodule umfaßt. Das Grundmodul umfaßt eine Steuerungs- und Signalauswerteeinrichtung mit einem Arbeitsspeicher und einem Programmspeicher zum Betrieb des Grundmoduls und zum Auswerten der von dem oder den jeweiligen Meßmodulen gelieferten Meßsignalen. Weiter umfaßt das Grundmodul eine Eingabe- und Bedienungseinrichtung, eine Ausgabe- und Anzeigeeinrichtung und eine Strom- und Spannungsversorgung. Die verschiedenen Meßmodule lassen sich mit Kabel mit unterschiedlichen Schnittstellen an dem Grundmodul verbinden.

Es ist daher Aufgabe der vorliegenden Erfindung ein vielseitig verwendbares, tragbares, netzunabhängiges Meßund Diagnosegerät zu schaffen, dessen Bedienung einheitlich und einfach ist und mit dem dennoch eine Vielzahl von unterschiedlichen Meßfunktionen realisiert werden können.

Die Lösung dieser Aufgabe erfolgt durch die Merkmale des Anspruchs 1.

Das erfindungsgemäße System besteht aus
1) einem Grundmodul, in dem sich die zentrale Recheneinheit mit Ein-/Ausgabeeinheiten, Daten-/Programmspeicher, Modulerkennungslogik und Power Management befinden;
2) verschiedenen anwendungsspezifischen Meßmodulen mit Meßsignalerzeugungs- und Erkennungselektronik
3) Die Meßmodule werden in das vorzugsweise länglich geformte und mit einer Hand bedienbare Grundgerät aufgesteckt und können einfach und ohne zusätzliche Hilfsmittel ausgewechselt werden.
4) Im Grundgerät befindet sich eine elektronische Schaltung, die ständig die Schnittstelle zu den Meßmodulen überwacht und durch eine Logikschaltung selbst erkennt, welches Meßmodul mit dem Grundgerät verbunden ist.
5) Die Schnittstellenlogik schaltet den Zentralrechner im Grundgerät automatisch in den Zustand der für den Betrieb mit dem ausgewählten Meßmodul erforderlich ist.
6) Modulerkennungslogik

Die Meß- bzw. Meßmodule besitzen eine eindeutig festgelegte Kennung die z.B. über eine hardwaregesteuerte Bitkombination oder über einen Bitcode, der einem nichtflüchtigen Speicher hinterlegt wird, von der Modulerkennungselektronik im Betrieb ständig abgefragt wird.

Die jedem Meßmodul zugeordnete Kennung ist auch im Programmspeicher der Zentraleinheit im Grundgerät hinterlegt.

Beim Verbinden eines Meßmoduls mit dem Grundgerät wird automatisch die Modulkennung abgefragt und mit den in der Zentraleinheit im Grundgerät abgespeicherten Kennungen verglichen.

Bei Übereinstimmung der Kennung eines Meßmoduls mit einer der in der Zentraleinheit dauerhaft hinterlegten Kennung wird der Zentralprozessor des Systems automatisch in den Betriebsmodus versetzt, der für die Datenverarbeitung des jeweiligen Meßmoduls erforderlich ist.

Durch die Verwendung eines einheitlichen Grundmoduls wird die Bedienung der unterschiedlichen Meßfunktionen vereinfacht, da zwangsweise eine einheitliche Bedienungsoberfläche vorliegt. An dieses Grundmodul lassen sich unterschiedliche Meßmodule für unterschiedlichste Funktionen anschließen. Durch die selbsttätige Erkennung der jeweils charakteristischen Kennung der Meßmodule wird im Grundmodul das jeweils geeignete Betriebs- bzw. Anwendungsprogramm ausgewählt.

Ein besonderer Vorteil der Erfindung besteht darin, daß nachträglich weitere Meßmodule hinzugefügt werden können.

Das erfindungsgemäße Meß- und Diagnosesystem läßt sich mit den verschiedensten Meßmodulen ausrüsten, so daß es für unterschiedlichste Zwecke geeignet ist. Hierzu zählen der medizinische Bereich mit Meßmodulen für Temperatur, Blutsauerstoffkonzentration, Bidirektionaler Ultraschalldoppler, Mehrkanaliges EKG, auch Langzeit-EKG, Blutdruckmessung, Spirometer, Stethoskop, Tonometer zur Bestimmung des Augendruckes, Laserscanner zur Bestimmung von Geometrie und Umfang einer Extremität etc., Oxymeter mit Hb-Detektor, Pulsoxymeter, Noninvasive Bilirubinbestimmung bei Neugeborenen, tp CO₂-Sensor, ph- und Redoxpotentialbestimmung, Blutgasbestimmung, Detektor zur Bestimmung von Glucose, -Lactat, -Glucamin und -Harnstoff, Detektor zur Bestimmung von Chlamydien, Vaginalmycosen und Kolibakterien, Photoakustischer Detektor zum Nachweis von Gaskonzentrationen in Miniproben, Laser-Velocity-System zur Detektion der Erythrozyten Geschwindigkeit in den Kapillaren.

In der Umweltschutztechnik sind Meßmodule für die Schadstoffmessung in den Abgasen von Kfz, in der Luft, im Wasser und im Erdreich möglich. Eine weitere Einsatzmöglichkeit ist die Überwachung der Produktqualität bei laufender Produktion. Durch geeignete Meßmodule wird die Konzentration bestimmter Stoffe in einem Produkt erfaßt.

Durch die kompakte Bauform und insbesondere durch ein stiftähnliches Gehäuse mit Dimensionen eines schlanken Diktiergeräts und durch eine entsprechende Anordnung der Bedienelemente wird erreicht, daß das erfindungsgemäße Gerät mit einer Hand gehalten und betätigt werden kann. Damit ist die Arbeit mit dem erfindungsgemäßen Gerät mit einer Hand möglich.

Durch die vorteilhafte Ausrüstung der Erfindung mit einer Diagnoselampe können Meßpunkte auf dem menschlichen Körper schneller und sicherer aufgefunden werden. Dies ist insbesondere dem ärtzlichen Notdienst von Vorteil. Außerdem ist es für den Arzt nicht notwendig eine separate Diagnoselampe mitzuführen, was insbesondere in Notfällen vorteilhaft ist.

Durch den Anschluß für ein elektronischen Stethoskops an die vorliegende Erfindung erübrigt sich der Einsatz eines herkömmlichen Stethoskops. Dies ist insbesondere in der Notfallmedizin vorteilhaft, da von dem Arzt wieder ein Gerät weniger mitgeführt werden muß. Außerdem verbessert es die Akzeptanz der Erfindung, da die Arbeit mit einem Stethoskop jedem Arzt vertraut ist.

Durch die Ausgestaltung der Erfindung mit einer elektronischen Speichereinrichtung ist es möglich Meßdaten zwischenzuspeichern, so daß sie mit nachfolgend aufgenommenen Meßdaten verglichen und weiterverarbeitet werden können. Damit ist beispielsweise auch möglich die zeitliche Entwicklung von Meßdaten darzustellen. Damit ist ein unmittelbarer optischer Vergleich von im zeitlichem Abstand aufgenommenen Meßdaten möglich. Auch können damit in vorteilhafter Weise im Körper eines Patienten symmetrisch angeordnete Gefäße nacheinander gemessen und anschließend gemeinsam dargestellt werden können.

Mittels der Echtzeituhr ist es zum einen möglich Zeitmessungen durchzuführen und zum anderen ist es möglich diese Zeitmessung sozusagen auf "Knopfdruck" Meßdaten zuzuordnen.

Durch die besonders vorteilhafte Ausgestaltung mit einer Diktiergerätefunktion (Anspruch 10 und 11) ist es dem Arzt möglich unmittelbar während der Messung zu der jeweiligen Messung Anmerkungen und sonstige Diagnosen zu diktieren. Auch Patientenname und sonstige erhebliche Daten lassen sich damit sprachlich fixieren. Damit wird die nachträgliche Auswertung der Meßdaten erheblich verbessert und auch vereinfacht. Durch die Zuordnung von Sprachaufzeichnung und/oder Datum und Uhrzeit wird die Diagnose auch sicherer und überprüfbarer. Die Kombination eines Diktiergeräts mit medizinischen Meß- und Diagnosegeräten ist ganz allgemein sinnvoll und nicht auf tragbare Geräte beschränkt.

Durch die Möglichkeit die mit dem erfindungsgemäßen Gerät gewonnen Informationen und Daten auf eine externe Vorrichtung zu übertragen, lassen sich weitergehende Auswertungen vornehmen. Auch lassen sich die Daten mit anderen Patientendaten in der externen Vorrichtung verknüpfen. Bei der externen Vorrichtung handelt es sich vorzugsweise um eine Adapterstation. Auf dieser Adapterstation lassen sich weitergehende Auswertungen der ermittelten Meßwerte durchführen. Die Adapterstation ist wiederum vorzugsweise mit eine Standard-Schnittstelle versehen, um sie mit einem Standard-PC oder einem Drucker verbinden zu können. Damit stehen die Daten auch für Krankenkassenabrechnungen zur Verfügung.

Die Verknüpfung mit anderen Patientendaten gestaltet sich besonders einfach, wenn das medizinische Meß- und Diagnosegerät und/oder die Adapterstation bzw. die externe Vorrichtung allgemein mit einer Lese/Schreibeinrichtung für Chipkarten ausgerüstet ist.

Durch Akkus, die in der Adapterstation aufgeladen werden wird auf einfache Weise eine autarke Betriebs- und Einsatzbereitschaft gewährleistet.

Durch eine Spannungsüberwachung wird verhindert, daß Meßdaten verloren gehen.

Durch das Vorsehen einer Quick-Start-Einrichtung ist beispielsweise eine Sofortdiagnostik in der Kreislaufüberwachung möglich, was insbesondere wieder in der Notfallmedizin von Vorteil ist.

Gemäß einer vorteilhaften Ausgestaltung der Erfindung ist die Steuerungs- und Signalauswerteeinrichtung so ausgelegt, daß über die Daten-/Signal-Schnittstelle das Betriebssystem des Geräts ohne Hardwareeingriff verändert und ausgetauscht werden kann. Damit ist es auf einfache Weise möglich nachträglich neue Meßmodule in das System einzubinden.

Die übrigen Unteransprüche beziehen sich auf weitere vorteilhafte Ausgestaltungen der Erfindung.

Weitere Einzelheiten, Merkmale und Vorteile der Erfindung ergeben sich aus der nachfolgenden Beschreibung anhand der Zeichnung.

Es zeigt:
Fig. 1 zeigt beispielhaft ein Blockschaltdiagramm einer bevorzugten Ausführungsform der Erfindung, und
Fig. 2 zeigt ein detailiertes Blockschaltdiagramm des Gesamtsystems. Meßmodule in Fig. 1 sind gekappselte und mit dem Grundgerät verbindbare elektromechanische und mit Sensoren ausgestattete Baugruppen, die verschiedene Funktionen erfüllen können. Beispiele hierfür sind:
   ◆ Messung der Blutströmung,
   ◆ Messung der Durchblutung in den oberen Hautschichten,
   ◆ Bestimmung des Sauerstoffgehalts im Blut,
   ◆ Bestimmung des Blutdrucks und der Herzfrequenz,
   ◆ EKG-Aufzeichnung,
   ◆ Temperaturmessung und Überwachung, usw.

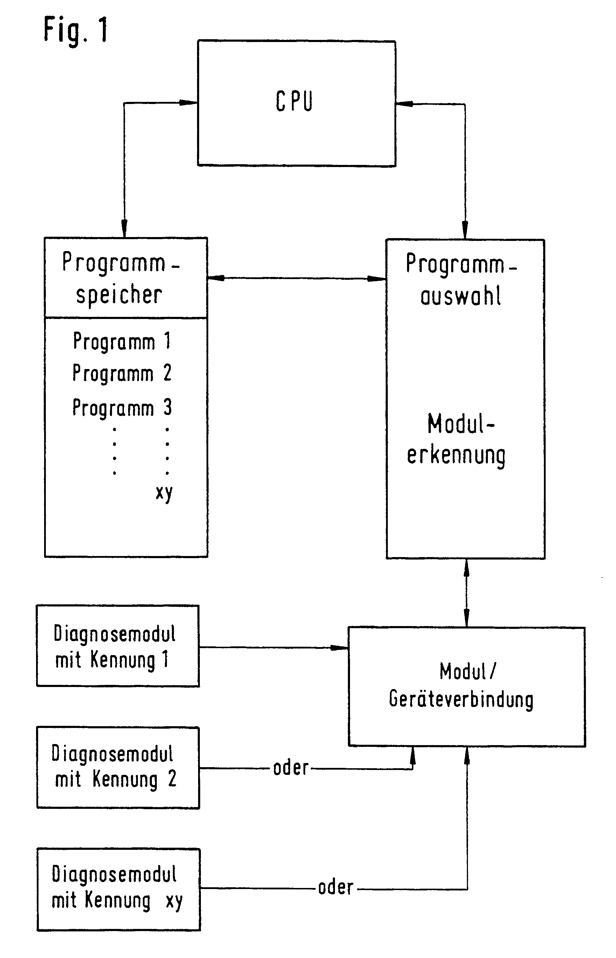

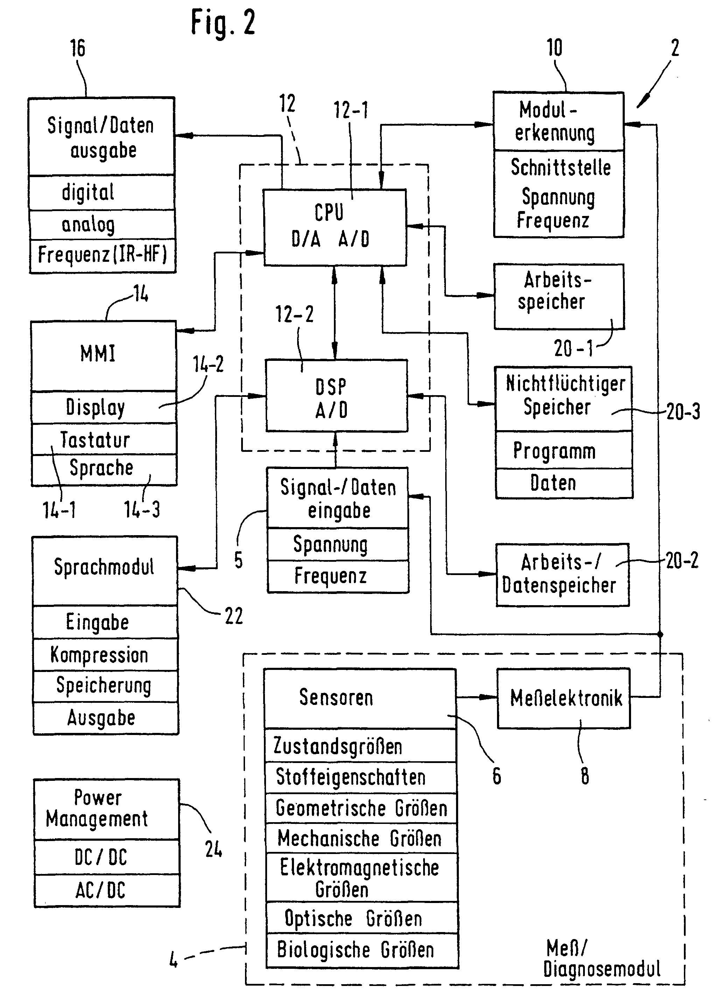

Fig. 2 zeigt ein detailliertes Blockschaltbild einer beispielhaften Ausführungsform der Erfindung. Das tragbare und netzunabhängige Meß- und Diagnosesystem gemäß Fig. 1 umfaßt ein Grundmodul 2 sowie ein beispielhaft dargestelltes Meßmodul 4. Das Meßmodul 4 umfaßt einen Sensor 6, der mit einer Meßelektronik 8 gekoppelt ist. Es werden Sensoren für unterschiedliche Gase, Flüssigkeiten, Temperatur, Farbe, Helligkeit, geometrische Größen, Stoffeigenschaften, mechanische Größen, elektromagnetische Größen, optische Größen und Eigenschaften und biologische Größen und Eigenschaften, usw. bereitgestellt.

Das jeweilige Meßmodul 4 ist über eine Signal/Daten-Schnittstelle 5 mit dem Grundmodul 2 verbunden. Das Grundmodul 2 umfaßt eine Modulerkennungseinrichtung 10, eine Steuerungs- und Signalauswerteeinrichtung 12 und eine Mensch-Maschine-Schnittstelle (MMI Man-Machine-Interface) 14. Die Mensch-Maschine-Schnittstelle 14 umfaßt eine Eingabetastatur 14-1, eine Anzeige 14-2 und ein Mikrofon 14-3. Des weiteren ist eine Daten/Signal-Schnittstelle 16 vorgesehen, mit der sich Daten und Signale von und zu dem tragbaren Meß- und Diagnosemodul übertragen lassen. Die Steuerungs- und Signalauswerteeinrichtung 12 umfaßt einen Mikroprozessor 12-1 mit D/A- und A/D-Wandler sowie einen zusätzlichen Prozessor 12-2 (DSP Digital Signal Processor). Mit dem zusätzlichen Prozessor 12-2 ist die Signal/Daten-Schnittstelle 5 verbunden, über die das jeweilige Meßmodul 4 angekoppelt wird. Mit der Steuerungs- und Signalauswerteeinrichtung 12 sind ein erster und zweiter Arbeitsspeicher 20-1 und 20-2 sowie ein nicht flüchtiger Speicher 20-3 für Programme und Daten verbunden. Die Modulerkennungseinrichtung 10 ist ebenfalls mit der Steuerungs- und Signalauswerteeinrichtung 12 verbunden.

Weiter ist ein Sprachmodul 22 mit der Steuerungs- und Signalauswerteeinrichtung 12 verbunden. Durch das Sprachmodul 22 kann zum einen das Meß- und Diagnosesystem mittels Sprachbefehl bedient werden, und zum anderen lassen sich zu verschiedenen Meßwerten sprachliche Informationen nach Art eines elektronischen Diktiersystems speichern. Die zu den jeweiligen Meßwerten gespeicherten sprachlichen Angaben können über Signal-Daten-Schnittstelle 16 ausgelesen werden.

Die Stromversorgung wird durch eine Stromversorgungs-Einheit 24 bereitgestellt. Die Stromversorgungs-Einheit umfaßt wiederaufladbare Akkumulatoren.

## Patentansprüche

1. Tragbares, netzunabhängiges Meß- und Diagnosesystem, mit
einer Mehrzahl von Meßmodulen (4) zur Messung und Speicherung physikalischer, chemischer und/oder biologischer Größen, und
einem Grundmodul (2),
das jeweils mit wenigstens einem der Mehrzahl der Meßmodule (4) lösbar verbindbar ist,
das eine Steuerungs- und Signalauswerteeinrichtung (12) zum Betrieb des Grundmoduls und zum Auswerten der von dem oder den jeweiligen Meßmodulen (4) gelieferten Meßsignalen umfaßt, wobei die Steuerungs- und Signalauswerteeinrichtung (12) mit einem Arbeitsspeicher (20-1, 20-2) und einem Programmspeicher (20-3) verbunden ist, und
das eine Eingabe- und Bedienungseinrichtung (14, 16), eine Ausgabe- und Anzeigeeinrichtung (14, 16), und eine Strom- und Spannungsversorgung (24) umfaßt,
wobei jedes Meßmodul (4) jeweils eine für das jeweilige Meßmodul (4) charakteristische Kennung aufweist,
und das Grundmodul (2) eine Modulerkennungseinrichtung (10) zum selbstätigen Erfassen der Kennung des jeweils mit dem Grundmodul (2) verbundenen Meßmoduls (4) aufweist,
**dadurch gekennzeichnet, daß** in dem Programmspeicher (20-3) der Steuerungs- und Signalauswerteeinrichtung (12) unterschiedliche Anwendungsprogramme zu den unterschiedlichen Meßmodulen (4) oder Kombinationen von Meßmodulen gespeichert sind, und
daß die Modulerkennungseinrichtung (10) das jeweilige Anwendungsprogramm zum Betrieb des Grundmoduls (2) und zum Auswerten der Meßdaten entsprechend der selbstätig erfaßten Kennung auswählt.

2. Vorrichtung nach Anspruch 1, **dadurch gekennzeichnet, daß** wenigstens das Grundmodul (2) eine kompakte Bauform aufeist, die langgestreckt und stiftähnlich ist, und daß das Grundmodul mit ein und derselben Hand gehalten und bedient werden kann.

3. Vorrichtung nach Anspruch 1 oder 2, **gekennzeichnet durch** wenigstens einen Datenspeicher (20-3) zum dauerhaften Abspeichern der erfaßten und aufbereiteten Meßsignale.

4. Vorrichtung nach wenigstens einem der vorhergehenden Ansprüche, **dadurch gekennzeichnet, daß** die Ausgabe- und Anzeigeeinrichtung (14) eine graphische und/oder eine akustische Anzeigeeinrichtung zum Anzeigen der von der Signalauswerteeinrichtung (12) erzeugten Signale aufweist.

5. Vorrichtung nach wenigstens einem der vorhergehenden Ansprüche, **dadurch gekennzeichnet, daß** das Grundmodul (2) und/oder ein Meßmodul (4) eine Diagnoselampe umfaßt.

6. Vorrichtung nach wenigstens einem der vorhergehenden Ansprüche, **dadurch gekennzeichnet, daß** die Ausgabeeinrichtung (16) einen Anschluß für eine elektronisches Stethoskop aufweist.

7. Vorrichtung nach wenigstens einem der vorhergehenden Ansprüche, **dadurch gekennzeichnet, daß** die Ausgabeeinrichtung (14) eine Einrichtung zum gleichzeitigen Darstellen von abgespeicherten und aktuellen Meßwerten in der Anzeigeeinrichtung aufweist.

8. Vorrichtung nach wenigstens einem der vorhergehenden Ansprüche, **gekennzeichnet durch** eine Echtzeituhr.

9. Vorrichtung nach wenigstens einem der vorhergehenden Ansprüche, **gekennzeichnet durch** eine Einrichtung (22) zum Aufzeichnen von Sprache.

10. Vorrichtung nach Anspruch 9, **dadurch gekennzeichnet, daß** die Sprachaufzeichnungsvorrichtung (22) mit der elektronischen Speichereinrichtung (20) gekoppelt ist.

11. Vorrichtung nach Anspruch 9 oder 10, **gekennzeichnet durch** eine Einrichtung (12) zum Zuordnen von Meßdaten zu Sprachaufzeichnungen und/oder Datum und Zeit aus der Echtzeituhr.

12. Vorrichtung nach wenigstens einem der vorhergehenden Ansprüche, **dadurch gekennzeichnet, daß** die Ausgabeeinrichtung (14, 16) eine Schnittstelleneinrichtung (16) aufweist, mittels der aktuelle oder abgespeicherte Meßdaten auf eine externe Vorrichtung übertragbar sind.

13. Vorrichtung nach Anspruch 12, **dadurch gekennzeichnet, daß** die Schnittstelleneinrichtung (16) eine Schnittstelle zur zur kontakthaften und/oder kontaktlosen Datenübertragung ist.

14. Vorrichtung nach wenigstens einem der vorhergehenden Ansprüche, **gekennzeichnet durch** eine Lese/Schreibeinrichtung für elektronische Datenträger, insbesondere für Chipkarten.

15. Vorrichtung nach wenigstens einem der vorhergehenden Ansprüche, **dadurch gekennzeichnet, daß** die Spannungs- und Stromversorgungseinrichtung (24) auswechselbare Akkus umfaßt.

16. Vorrichtung nach wenigstens einem der vorhergehenden Ansprüche, **gekennzeichnet durch** eine Einrichtung zum Überwachen der Spannungs- und Stromversorgung (24) und zum automatischen Abspeichern der aktuellen Meßdaten und/oder Betriebsparamater, falls die Spannung der Spannungs- und Stromversorgung (24) einen bestimmten Grenzwert unterschreitet.

17. Vorrichtung nach wenigstens einem der vorhergehenden Ansprüche, **gekennzeichnet durch** eine Quick-Start-Einrichtung **durch** die das Gerät unmittelbar nach Inbetriebnahme einsatzbereit ist.

18. Vorrichtung nach wenigstens einem der vorhergehenden Ansprüche, **gekennzeichnet durch** eine Adapterstation, wobei zwischen Grundmodul (2) und Adapterstation drahtlos Daten austauschbar sind.

19. Vorrichtung nach Anspruch 18, **dadurch gekennzeichnet, daß** die Adapterstation eine Lese/Schreibeinrichtung für elektronische Datenträger, insbesondere für Chipkarten umfaßt.

20. Vorrichtung nach wenigstens einem der vorhergehenden Ansprüche, **dadurch gekennzeichnet, daß** die für das jeweilige Meßmodul (4) charakteristische Kennung ein Bitcode ist, der im Programmspeicher (20-3) des Grundmoduls dauerhaft abgespeichert ist, und daß das jeweilige Anwendungsprogramm durch Vergleich der abgespeicherten Kennungen mit der selbstätig durch die Modulerkennungseinrichtung (10) erfaßten Kennung ausgewählt wird.

21. Vorrichtung nach Anspruch 20, **dadurch gekennzeichnet, daß** die Steuerungs- und Signalauswerteeinrichtung (12) die Kennung auf Interruptbasis kontinuierlich abfragt.

22. Vorrichtung nach wenigstens einem der vorhergehenden Ansprüche, **dadurch gekennzeichnet, daß** daß Grundmodul (2) eine Schnittstelle (18) zum Verändern des Betriebssystems ohne Hardwareeingriff umfaßt.

## Claims

1. Portable, mains-independent measurement and diagnosis system, having a plurality of measurement modules (4) for measuring and storing physical, chemical and/or biological parameters and a base module (2) which in each case can be connected removably to at least one of the plurality of measurement modules (4) comprising a control and signal evaluation facility (12) for operating the base module and for the evaluation of the measurement signals delivered by the respective measurement module or measurement modules (4), **characterized in that** the control and signal evaluation facility (12) is connected to a work memory (20-1, 20-2) and a program memory (20-3), comprising an input and operating facility (14, 16), an output and display facility ( 14, 16) and a power and voltage supply (24), where each measurement module (4) having an identification characteristic of the respective measurement module (4), where the base module (2) comprises a module recognition facility (10) for automatic recording of the identification of the respective measurement module (4) connected to the base module (2), **characterized in that** different application specific programs are stored for each or combinations of measurement modules (4) in the operating memory (20-3) of the control and signal evaluating facility (12), and **in that** the module recognition facility (10) selects the respective application program for operating the base module (2) and for evaluating the measurement data in accordance with the automatically recorded identification.

2. Device according to claim 1, **characterized in that** at least the base module (2) has a compact structure which is elongate and pen-shaped, and **in that** the base module can be held and operated with one and the same hand.

3. Device according to claim 1 or 2, **characterized by** at least one data memory (20-3) for permanently storing the recorded and prepared measurement signals

4. Device according to at least one of the preceding claims, **characterized in that** the output and display facility (14) has a graphic and/or an acoustic display facility for displaying the signals generated by the signal evaluation facility (12)

5. Device according to at least one of the preceding claims, **characterized in that** the base module (2) and/or a measurement module (4) comprises a diagnosis light.

6. Device according to at least one of the preceding claims, **characterized in that** the output facility (16) has a connection for an electronic stethoscope.

7. Device according to at least one of the preceding claims, **characterized in that** the output facility (14) has a device for simulaneously representing stored and current measurement values in the display facility.

8. Device according to at least one of the preceding claims, **characterized by** a real-time clock.

9. Device according to at least one of the preceding claims, **characterized by** a facility (22) for recording speech.

10. Device according to claim 9, **characterized in that** the speech- recording device (22) is coupled to the electronic memory facility (20)

11. Device according to claim 9 or 10, **characterized by** a facility (12) for assigning measurement data to speech recordings and/or date and time from the real-time clock.

12. Device according to at least one of the preceding claims, **characterized in that** the output facility (14, 16) has an interface facility (16) by means of which current or stored measurement data can be transferred to an external device.

13. Device according to claim 12, **characterized in that** the interface facility (16) is an interface for contact and/or contactless data transfer.

14. Device according to at least one of the preceding claims, **characterized by** a read/write facility for electronic data carriers, in particular for chip cards.

15. Device according to at least one of the preceding claims, **characterized in that** the voltage and power supply facility (24) includes replaceable batteries.

16. Device according to at least one of the preceding claims, **characterized by** a facility for monitoring the voltage and power supply (24) and for automatically storing the current measurement data and/or operating parameters if the voltage of the voltage and power supply facility (24) drops below a defined limit value.

17. Device according to at least one of the preceding claims, **characterized by** a quick-start facility by means of which the appliance is ready for use directly after being brought into service.

18. Device according to at least one of the preceding claims, **characterized by** an adapter station, with wireless transfer of data between base module (2) and adapter station.

19. Device according to claim 18, **characterized in that** the adapter station comprises a read/write facility for electronic data carriers, in particular for chip cards.

20. Device according to at least one of the preceding claims, **characterized in that** the identification characteristic of the respective measurement module (4) is a bit code which is stored permanently in the program memory (20-3) of the base module, and **in that** the respective application program is selected by comparing the stored identifications with the identification automatically recorded by the module recognition facility (10)

21. Device according to claim 20, **characterized in that** the control and signal evaluation facility (12) continuously asks for the identification on interrupt basis.

22. Device according to at least one of the preceding claims, **characterized in that** the base module (2) comprises an interface (18) for changing the operating system without hardware involvement.

## Revendications

1. Système de mesure et de diagnostic portable, fonctionnant indépendamment du secteur, comportant plusieurs modules (4) pour la mesure et l'enregistrement de valeurs physiques, chimiques et/ou biologiques ainsi qu'un module de base (2) pouvant être relié de manière amovible à au moins l'un des différents modules de mesure (4), comprenant un dispositif de commande et d'évaluation des signaux (12) pour l'exploitation du module de base et pour l'évaluation des signaux de mesure fournis par le(s) module(s) de mesure respectif(s) (4), sachant que le dispositif de commande et d'évaluation des signaux (12) est relié à une mémoire de travail (20-1, 20-2) et à une mémoire programme (20-3), et qui comporte un dispositif de saisie et de manoeuvre (14, 16), un dispositif de sortie et d'affichage (14, 16) ainsi qu'une alimentation en courant et en tension (24), chaque module de mesure (4) disposant d'une identification typique pour le module de mesure (4) respectif, et le module de base (2) comportant un dispositif de reconnaissance de module (10) pour la saisie autonome de l'identification des modules de mesure respectifs (4) reliés au module de base (2), **caractérisé en ce que** différents programmes d'application appartenant aux différents modules de mesure (4) ou aux combinaisons des modules de mesure sont sauvegardés dans une mémoire programme (20-3) du dispositif de commande et d'évaluation des signaux, et **en ce que** le dispositif de reconnaissance des modules (10) choisit le programme d'application nécessaire pour le fonctionnement du module de base(2) ainsi que pour l'évaluation des données de mesure en fonction de l'identification saisie de façon autonome.

2. Dispositif selon la revendication 1, **caractérisé en ce qu'**au moins le module de base (2) adopte une construction longiligne et similaire à celle d'une tige, et que le module de base peut être tenu et manoeuvré d'une seule main.

3. Dispositif selon la revendication 1 ou 2, **caractérisé par** au moins une mémoire de données (20-3) permettant de sauvegarder de façon permanente les signaux saisis et traités.

4. Dispositif selon au moins l'une des revendications qui précèdent, **caractérisé en ce que** le système de sortie et d'affichage (14) comporte un dispositif de signalisation graphique et/ou acoustique des signaux générés par le dispositif d'évaluation des signaux (12).

5. Dispositif selon au moins l'une des revendications qui précèdent, **caractérisé en ce que** le module de base (2) et/ou l'un des modules de mesure (4) dispose d'une lampe de diagnostic.

6. Dispositif selon au moins l'une des revendications qui précèdent, **caractérisé en ce que** le dispositif de sortie (16) dispose d'une prise pour un stéthoscope électronique.

7. Dispositif selon au moins l'une des revendications qui précèdent, **caractérisé en ce que** le dispositif de sortie (14) comporte un dispositif pour l'affichage simultané des valeurs de mesure sauvegardées et actuelles sur le dispositif d'affichage.

8. Dispositif selon au moins l'une des revendications qui précèdent, **caractérisé par** une horloge en temps réel.

9. Dispositif selon au moins l'une des revendications qui précèdent, **caractérisé par** un dispositif (22) d'enregistrement vocal.

10. Dispositif selon la revendication 9, **caractérisé en ce que** le dispositif d'enregistrement vocal (22) et le dispositif de mémoire électronique (20) sont raccordés ensemble.

11. Dispositif selon la revendication 9 ou 10, **caractérisé par** un dispositif (12) d'affectation de données de mesure à des enregistrements vocaux et/ou à la date et à l'heure fournies par l'horloge en temps réel.

12. Dispositif selon au moins l'une des revendications qui précèdent, **caractérisé en ce que** le dispositif de sortie (14, 16) est muni d'un dispositif d'interface (16) par lequel les données actuelles ou sauvegardées peuvent être transférées vers un dispositif externe.

13. Dispositif selon la revendication 12, **caractérisé en ce que** le dispositif d'interface (16) constitue une interface pour la transmission des données avec et/ou sans contact.

14. Dispositif selon au moins l'une des revendications qui précèdent, **caractérisé par** un dispositif de lecture/d'écriture de supports de données électroniques, en particulier pour des cartes à puce.

15. Dispositif selon au moins l'une des revendications qui précèdent, **caractérisé en ce que** le dispositif d'alimentation en tension et en courant (24) est équipé de batteries amovibles.

16. Dispositif selon au moins l'une des revendications qui précèdent, **caractérisé par** un dispositif de surveillance de l'alimentation en tension et en courant (24) et de sauvegarde automatique des données de mesure actuelles et /ou des paramètres d'exploitation si la tension de l'alimentation en tension et en courant (24) tombe sous une certaine valeur limite.

17. Dispositif selon au moins l'une des revendications qui précèdent, **caractérisé par** un dispositif Quick-Start (démarrage rapide) qui permet à l'appareil d'être opérationnel aussitôt après la mise en marche.

18. Dispositif selon au moins l'une des revendications qui précèdent, **caractérisé par** un poste d'adaptation, des données pouvant être échangées sans câble entre le module de base (2) et ce poste d'adaptation.

19. Dispositif selon la revendication 18, **caractérisé en ce que** le poste d'adaptation comporte un dispositif de lecture/d'écriture pour des supports de données électroniques et en particulier pour des cartes à puce.

20. Dispositif selon au moins l'une des revendications qui précèdent, **caractérisé en ce que** l'identification caractéristique pour le module de mesure (4) respectif est un code binaire qui est sauvegardé en permanence dans la mémoire programme (20-3) du module de base, et **en ce que** le programme d'application correspondant est sélectionné de manière autonome par la comparaison entre l'identification sauvegardée et l'identification saisie par le dispositif de reconnaissance de module (10).

21. Dispositif selon la revendication 20, **caractérisé en ce que** le dispositif de commande et d'évaluation des signaux (12) interroge en permanence l'identification sur la base des interruptions.

22. Dispositif selon au moins l'une des revendications qui précèdent, **caractérisé en ce que** le module de base (2) comporte une interface (18) qui permet de changer de système d'exploitation sans intervention sur le matériel.
